# EUROPEAN PATENT SPECIFICATION

(11) **EP 1 165 159 B1**
(45) Date of publication and mention of the grant of the patent: **26.03.2008**
(21) Application number: 01910272.2
(22) Date of filing: 07.02.2001
(51) Int. Cl.: A61M 1/38, B01D 15/08, B01J 20/22, C07H 3/06

(54) **OLIGOSACCHARIDE SUPPORTS FOR E.G. REMOVAL OF ANTIBODIES FROM BLOOD**
OLIGOSACCHARIDENTRÄGER, BEISPIELSWEISE FÜR DIE ENTFERNUNG VON ANTIKÖRPERN AUS DEM BLUT
SUPPORT OLIGOSACCHARIDE DESTINE NOTAMMENT A RETIRER DES ANTICORPS DU SANG

(30) Priority: 08.02.2000 SE 0000430; 16.05.2000 SE 0001833; 28.06.2000 SE 0002462; 24.11.2000 SE 0004343
(43) Date of publication of application: 02.01.2002
(73) Proprietor: Glycorex Transplantation AB, 223 70 Lund (SE)
(72) Inventor: NILSSON, Kurt, S-226 53 Lund (SE)
(74) Representative: Henriksson, Dan Ragnar Mikael
(86) International application number: PCT/SE2001/000241
(87) International publication number: WO 2001/058510

(56) References cited:
- EP-A2- 0 371 636
- US-A- 4 137 401
- US-A- 4 238 473
- US-A- 5 541 294
- US-A- 5 651 968
- US-A- 5 695 759
- L. BLOMBERG ET AL.: 'Improved removal of anti-A and anti-B antibodies from plasma using blood-group-active haptens' VOX SANG vol. 65, 1993, pages 126 - 135, XP002941315
- PALIAKKARA L. JAISON ET AL.: 'Rapid isolation of human plasma anti-alpha-galactoside antibody using sugar-specific binding to guar galactomannan or agarose' INDIAN JOURNAL OF BIOCHEMISTRY & BIOPHYSICS vol. 29, June 1992, pages 266 - 270, XP002941314

## Description

The present invention relates to a column for treatment of whole blood or blood plasma, to a method for filling said column, to a method for extracorporeal removal of blood group A and blood group B antibodies from whole blood or blood plasma, to use of said column for said extracorporeal removal, to a saccharide-spacer-O-matrix product and to use thereof in said column during said method for extracorporeal removal.

In one aspect the present invention relates to a column for treatment of whole blood or blood plasma, said column having the characteristics disclosed in independent claim 1.

In another aspect the present invention relates to a method for filling said column, wherein said method comprises the steps disclosed in independent claim 20.

In still another aspect the present invention relates to a product represented by the structure: saccharide-spacer-O-matrix, wherein said product has the characteristics disclosed in independent claim 21.

In a further aspect the present invention relates to a method for extracorporeal removal of blood group A and blood group B antibodies from whole blood or blood plasma, wherein said whole blood or blood plasma is brought to pass through said column.

In still a further aspect the present invention relates to use of said column for extracorporeal removal of blood group A and blood group B antibody from whole blood or blood plasma.

In still a further aspect the present invention relates to use of said product in said column for extracorporeal removal of blood group A and blood group B antibodies from whole blood or blood plasma.

Further features of the different aspects appear from the subsequent dependent claims.

A material containing so-called matrix to which saccharide has been bound via a spacer is described below.

The active part of the material according to the invention, contains at least one Saccharide part which has been bound via a spacer to a Matrix according to:

Saccharide-spacer-Matrix.

The Matrix consists in general of a polymer, plastic, or a polysaccharide, and can bind a large number of Saccharine-spacer units.

Saccharide symbolizes saccharide which has a biological or other affinity to another molecule, protein virus or cell. Saccharide can consist of a glycoprotein, a neoglycoprotein, a glycopeptide or a glycosylated amino acid, a glycolipid, or a part, a fragment or a modified variant thereof, or another biologically active di- or trisaccharide or higher oligosaccharide substance.

A few non-limiting examples of biologically active saccharides, spacer and Matrix which can be used according to the invention, are given below.

The active part of the Material consists, as a non-limiting example, of for example either.
1. Blood group AO(CH₂)ₙPhNH-CO-(CH₂)ₘNH-CH₂-CH(OH)-CH₂-O-Matrix
   or:
2. Blood group B-O(CH₂)ₙPhNH-CO-(CH₂)ₘNH-CH₂-CH(OH)-CH₂-O-Matrix
where Matrix denotes e.g. a plastic or a polysaccharide, for example cross-linked agarose, specifically of the type Sepharose^{R} Fast Flow, where -O(CH2)nPhNH-CO-(CH2)MNH-CT42-CH(OH)-CH.2- is spacer, according to the invention, to separate the Saccharide, in above examples blood group determinant A- and B-, respectively, from the Matrix,
where n and m, respectively, is an integer, n is for example one of 0, 1,2, 3 or 4, and m is for example 1, 2, 3, 4, 5, 6 or 7, and where the linkage between -O- and Matrix is formed between -O- and for example a carbon atom in the Matrix.

Saccharide-spacer, for example Blood group A-O(CH₂)ₙPhNH-CO-(CH₂)ₘNH- and Blood group B-O(CH₂)ₙPhNH-CO-(CH₂)ₘNH- respectively, is below called (the) ligand. The Matrix has a large number of bound molecules of ligand. Examples of bound amount of ligand is 0.01, 0.1, 1,2,3,4.5. 6,7,8,9,10,11,12,13,14,15,16,17,18,19,20 mmole per liter of Matrix, or an amount of mmole which is between two of the above given values per liter of Matrix. Per liter Matrix here means the volume occupied by the ready-to-use Matrix product.

A combination of two or more different saccharides can be used according to the invention, for example as non-limiting example, a combination of Blood group A-O(CH₂)ₙPhNH-CO-(CH₂)ₘNH-, and Blood group B-O(CH₂)ₙPhNH-CO-(CH₂)ₘNH-, where both ligands in this example are bound to Matrix.

A non-limiting example of a preferred variant of product 1 above is:

### 1.a. GalNAcα1-3(Fucα1-2)Galβ-O(CH₂)₂PhNH-CO-(CH₂)₅NH-CH₂-CH(OH)-CH₂-O-Matrix

This type of product can be produced by reaction between for example GalNAcα1-3(Fucα1-2)Galβ-O(CH₂)₂PhNH₂ and for example NHS-activated Sepharose^{R} 4FF, where the latter is commercially available, or cross-linked agarose or other Matrix with corresponding properties. The reaction conditions are chosen by the expert and does not limit the scope of the invention. Other examples are for example product containing a in the same manner bound, higher oligosaccharide than the A-trisaccharide in the above example, which contains the A-determinant terminally, for example A-determinant of type 1, 2, 3 or 4. The trisaccharide derivative GalNAcα1-3(Fucα1-2)Galβ-O(CH₂)₂PhNH₂ and other saccharide derivatives mentioned in this application can be produced with different chemical and/or biochemical methods and this do not limit the scope of the invention.

Further examples of product 1 is Product where a combination of 1.a. and one or more of mentioned blood group A variants, are bound via the same type of spacer as shown above to Matrix, or via a different type of spacer,

A non-limiting example of a preferred variant of product 2 above is:

### 2.b. Galα1- 3(Fucα1-2)Galβ-O(CH₂)₂PhPhNH-CO-(CH₂)₅NH-CH₂-CH(OH)-CH₂-O-Matrix

This type of product can be produced by reaction between for example Galα1-3(Fucα1-2)Galβ-O(CH₂)₂PBNH₂ and for example NHS-activated Sepharose^{R} 4FF, where the latter is commercially available. The reaction conditions are chosen by the expert and does not limit the scope of the invention. Other examples are for example product containing a in the same manner bound higher oligosaccharide, which contains the B-determinant terminally, for example B-determinant of type 1, 2, 3 or 4. Further examples of product 2 are Material where a combination of 2.b. and one or more of mentioned blood group B variants, are bound via the same type of spacer as above to Matrix, or via a different type of spacer.

Instead of the -O(CH₂)₂PhNH- group in the formulas above, another suitable Spacer or part of Spacer can be used, as for example -O(CH₂)ₙNH- or for example N(Ac)-(CH₂)ₙNH- (Ac = Acetyl group; n is an integer, for example 1, 2, 3, 4, 5, 6, or 7 or higher), or another aliphatic compound, or another aromatic compound.

The saccharide, for example the blood group A- or B- determinant, can also be bound, directly or indirectly, to an oligomeric substance acting as Spacer, or part of Spacer, as for example a mono-, di-, or higher oligosaccharide or polysaccharide, peptide, for example a peptide consisting of amide bound glycine and glutamic acid residues, for example Gly-(Glu-Gly)n-Glu, where n is an integer between for example 1 and 20. In this manner the Saccharide-spacer consists of 1, 2, 3, 4, 5, 6, 7, 8, 9, or 10 or more saccharide units bound to each oligomeric substance or peptide.

The linkage between the saccharide and the peptide can for example be formed via O-glycvsidically bound -O(Cli₂)₂PhNH- group (see for example formulas 2.a- and 2.b. above), or via for example O-glycosidically bound -O(CH₂)ₙNH-, (where n is an integer for example 1,2,3,4,5,6,7 or higher), where NH- is bound via an amide linkage (NH-CO) to the carboxyl group on the side-chain of the Glu-residues in the peptide. -O in -O(CH₂)₂PhNH- and in -O(CH₂)ₙNH-, respectively, is then bound glycosidically to the Saccharide.

The peptide can first have been coupled to Matrix, for example NHS-activated Sepharose^{R} 4FF via the α-amino-group on the peptide, and thereafter can the saccharide be bound to the peptide via -O(CH₂)₂PhNH-, or for example -O(CH₂)ₙNH-, to the carboxyl group on the Glu-residues in the peptide. This linkage between saccharide and Glu-residues can be achieved by for example carbodiimide-(for example EDC-) mediated coupling, or by for example succinimide-mediated coupling. The saccharide-spacer can herewith be added to the reaction mixture in for example a desired molar excess in relation to the amount of moles of peptide, e.g. in a molar excess of 2, 3, 4, 5, 6, 7, 8, 9 or 10 times excess or more. These and other reaction conditions are chosen by the expert and do not limit the scope of the invention. Non-limiting examples of in this manner bound amount of Saccharide is 0.01, 0.1, 1, 2, 3, 4, 5, 6, 7, 8, 9, 10, 11, 12, 13, 14, 15, 16, 17, 18, 19, 20 mmole of ligand per liter of Matrix. Per liter of Matrix means here the volume occupied by the ready to use Matrix product.

Another example of peptide is as above, but containing at least one Lysine residue, where the ε-amino group in the Lysine residue of the peptide is used for covalent coupling to Matrix, for example NHS-activated Sepharose^{R} 4FF, with subsequent coupling of, for example, Saccharide-O(CH₂)₂PhNH-, or of for example Saccharide-O(CH₂)ₙNH-, to coupled peptide according to what has been describe above. Other linkages can be used according to the invention.

An advantage of using oligomeric ligands, as above mentioned examples of saccharide-peptide ligands, is that a stronger binding often can be achieved to that which is desired to separated by the product, for example of antibodies towards blood group determinant or of other proteins, viruses or cells, and that therewith a more efficient product can be obtained as compared with non-oligomeric ligand.
As a non-limiting example of one preferred variant of Material according to the invention and as a non-limiting example of its production may be mentioned the coupling of ca 3, 1.5 and 1 µmol, respectively, of the peptide Ac-Lys-(s-amino)-Gly-GIu-Gly-Glu-Gly-Glu-GIy-Glu-Gly-Glu-Gly-Glu-Gly-amide via its ε-amino group, to 2 ml ofNHS-activated Sepharose 4 FP, from Pharmacia-Biotech, at pH 7.5 (0.2 M sodium fosfate buffer + 0.5 M NaCl) under 4 h at room temperature followed by 0.3 M Tris-HCl, pH 8, at room temp.; over night. The gel is washed with Tris-buffer and 0.1. M MES, pH 4.7, which gave Peptide-Sepharose 4FF. Saccharide-spacer, as for example Galα1-3Galα-OPhNH₂, ca 25 mikromole, dissolved in 0.1 M MES-bufFert, pH 4.7, was added to a solution of 48 mg EDC ((1-Ethyl-3-(3-Dimethylatninopropyl)-Carbodilmide)), to which was added 2 ml of Peptide-Sepharose 4FF, the mixture was incubated under 4 h at room temperature, after which it was washed with Tris-HCl, pH 8, 0.1 M acetate- and 0.1 M sodium fosfate buffer, respectively. The gels were tested for binding of antibodies, in the example of anti-Galα1-3Gal antibodies and showed a better binding of IgM antibodies as compared with the same amount of Sackarid-spacer-Sepharose 4FF, obtained by coupling of the corresponding amount of Galα1-3Galα-OPhNH-, but without peptide, directly to NHS-activated Sepharose 4FF. Other saccharide derivatives than Galα1-3Galα-OPhNH₂ can be used according to the invention, such as for example GaINAcα1-3(Fucal-2)Galβ-O(CH2)₂PhNH₂ or Galα1-3(Fucα1-2)Galβ-O(CH₂)₂PhNH₂
The saccharide-peptide conjugate can for example first be prepared by using for example the, ε-BOC-derivative (BOC = tert-butyloxycarbonyl group, situated on the ε-amino group of the Lysine residue) of for example the peptide in the above mentioned example. The Saccharide-spacer-peptide konjugat is then first formed by for example EDC-mediated reaction between amino groups on the saccharide-spacer and the carboxyl groups on the peptide, the resulting conjugate can be purified by for example Sephadex chromatography, the BOC group can be eliminated by for example trifluoroacetic acid reaction according to standard conditions for peptide chemistry, and the conjugate can be coupled for example in the same manner as describe above via the e-amino group of the lysine residue to the Matrix, for example NHS-activated Sepharose 4 FF.

As another non-limiting example of peptide can be mentioned peptide consisting of amide bound Gly and Lys units, for example Gly-(Lys-Gly)n-Gly, where n is an integer between for example 1 and 20. In this case can for example the peptide be bound to the Saccharide via amino groups on the peptide, a N-glycosidic linkage is formed between the reducing end on the Saccharide and the ε-amino group on the Lysine residue(s), and the Saccharide-peptide can be coupled to the Matrix via for example either the remaining amino group(s) on the peptide to for example NHS-activated Sepharose as describe above, or via for example the terminal COO-group on the peptide and amino groups on amino group containing Matrix, for example aminohexyl-Sepharose (by for example carbodiimide or succinimide coupling according to examples given above). The N-glycosidic linkage can be stabilised by acetylation under standard conditions, for example before coupling to the Matrix, e.g. NHS-activated Sepharose 4 FF. In the same manner as for the Gly-Glu-peptide above can also an aliphatic or aromatic spacer be used to bind the Saccharide to the Lysine residues of the peptide, but in this case is, for example, glycosidically bound groups of the type -O(CH₂)₂PhCOO-,or for example O(CH₂)ₙCOO-, used for carbodiimide- or succinimide-mediated coupling between Saccharide and Lysine residues in the peptide.

The coupling to the peptide can also be carried out by first coupling the Saccharide part to the amino acid and thereafter form the peptide linkages.

Further examples of Ligand according to the invention, is to use a protein or a polysaccharide as Spacer, or part of Spacer, between Saccharide and Matrix. Here for example a protein, such as serum albumin, or a polysaccharide, such as dextran, is used. The Saccharide can then first be coupled to the protein, or to the polysaccharide, which then is coupled to the Matrix. The same type of chemistry as exemplified above can, as non-limiting examples, be used to achieve the linkages between Saccharide, protein, or polysaccharide, and Matrix. This do not limit the scope of the invention, and the condition; are chosen by the expert.

To use a peptide, protein or polysaccharide according to what have exemplified above, can in some cases be an advantage to increase the ability of the Material to bind protein, and thereby increase the efficiency of the product according to the invention.

As another example of Matrix can be mentioned the filters which are used for plasma separation. These can be chemically modified with standard technique and be used for coupling of oligomeric ligand or of non-oligomeric ligand mentioned in this description. In this manner is achieved a product which can be used for specific removal of proteins in connection with bloodplasma separation, for example antibodies directed towards Galα1-3Gal and other so called xeno antigens in connection with xenotransplantation.

In a variant of the invention, the product in addition contains a Tris structure according to the following non-limiting example: (HOCH₂)₃C-NH-CO-(CH₂)₅NH-CH₂-CH(OH)-CH₂-O-Matrix
where (HOCH₂)₃C-NH- is a so called Tris-group. This product can be made by reaction between Tris-HCl and for example NHS-activated Sepharose 4 FF in which case Matrix above is Scpharose 4 FF.

In the production of the product according to the invention can for example be used commercially available activated Matrix, for example so called NHS-activated Sepharose^{R} 4 Fast Flow (NHS- is an abbreviation of N-hydroxysuccinimide; this variant of agarose is relatively strongly cross-linked, commercially available), which is present in the form of practically spherical particles. The particle size is chosen in, for example, the interval 45 - 165 µm. This activated Matrix can be used for covalent binding of for example, Blood group A-O(CH₂)ₙPhNH₂-, to give product I.a. above, and of Blood group B-O(CH₂)ₙPhNH₂, which give product 2.b. above, respectively, at, as non-limiting and typical example, pH 7.5 or pH 8.0, in buffer, for example 0.1 M sodium phosphate as non-limiting example, under for example 1, or 2 hours or for 20 hours, and in the example at room temperature. The material is washed for example on a glass filter or under other conditions, for example sterile conditions, with for example buffer and is subsequently treated with for example Tris-HCl buffer to react any remaining reactive groups. The expert chooses the conditions for the reactions and this do not limit the scope of the invention. See also the above given example in connection with the preparation of Galα1-3 Gal-Peptide-Sepharose 4FF.

In the production of the product according to the invention can, as another example, be used so-called epoxy-activated Sepharose^{R} 4 Fast Flow, to which is covalently bound, for example Blood group A-O(CH₂)ₙPhNH-CO-(CH₂)ₘNH-, or to which is covalently bound Blood group B-O(CH₂)ₙPhNH-CO-(CH₂)ₘNH-,
where n och m are specified above as are Blood group A and Blood group B, respectively.

As has been mentioned above, a combination of ligands can also be covalently bound the Matrix.

The products can be used, for example, for extra-corporal removal of blood group A- and blood group B-antibodies, respectively, e.g. for purification of blood, or for example, before a transplantation, for example over the blood group barrier. The product can be used in general for different types of transplantation as a part of the treatment of the recipient before and during, and eventually after the transplantation. This to be able to solve the problem of blood group incompatibility between donor and recipient. The product can for this purpose be filled into a column housing with in- and outlet for passage through the column of for example blood plasma, or whole blood, from the patient who shall be transplanted or who is undergoing a transplantation procedure. The use of the product is therefore not restricted to for example, blood group incompatible transplantation, but can also be used, for example, for blood group compatible transplantation, to minimize problems in connection with donor and recipient of the same blood group, but of different blood group subgroups, for example A1, A2 etc.

Other non-limiting examples of Saccharide according to the specific examples 1 och 2 above, are structures where the saccharide part consists of Galα1-3Galα-, Galα1-3Galβ-, Galα1-3Galβ1-4Glcβ-, Galα1-3Galβ1-4GlcNAcβ-, Gala1-3Galβ1-4GlcNAcβ1-3Galβ1-4Glcβ-, or of oligomeric ligands, such as for example (Galα1-3Galα-)n-, (Galα1-3Galβ-)n-, (Galα1-3Galβ1-4Glcβ-)n-, (Galα1-3Galβ1-4GlcNAcβ-)n-, (Gαlα1-3Gαlβl-4GlcNAcβ1-3Galβ1-4Glcβ-)n-, or (Ga1α1-3Galα- spacer)n-, (Gαlα1-3Gαlβ-spacer)n-, (Galαl-3Gα1β1-4Glcβ-spacer)n-, (Gαlα1-3Galβ1-4GlcNAcβ-spacer)n-, (Gαlα1-3Galβ1-4GlcNAcβ 1-3Galβ 1-4Glcβ-spacer)n-,
where n is an integer larger than 1. As mentioned above for other saccharides, different spacers can be used in connection with above mentioned saccharides. Non-limiting examples of spacer have been given above, for example of non-oligomeric, oligomeric, and of polymeric type, respectively.
These structures can be of interest to be used in for example a column or in a plasmafilter, for example before and after xenotransplantation to reduce so called xeno-antibodies from the patient's blood (whole blood column) or plasma.

Other carbohydrate structures active towards other antibodies, for example antibodies against cancer-antigens, for example prostate-, breast-, intestine-, or skin cancer, can be used to form Product according to the invention. This can be of interest for example to isolate antibodies from blood or plasma against said antigen and after elution from the Product, be used for treatment of said cancer diseases, or to produce reagents, or for example, to remove an excess of antibody derivatives from blood or plasma in immunotherapy of cancer. Other carbohydrate structures specific for e.g. toxins, virus and/or bacteria, can also be used to form Product according to the invention. These Products can be used to, for example, purify or eliminate virus and/or bacteria from e.g- whole blood or plasma or from other materials, for example food products or from water.

A combination of two or more different ligands (Saccharide-spacer) bound to Matrix, can be used in the Product according to the invention. The saccharides can then be different, and/or the spacer can be different.

The product according to the invention, allows for example a combination of high flow rate (for example in the interval 20-60 ml/min), minimal drop in pressure over the column, and a good binding capacity also of molecularly larger proteins, for example antibodies, such as IgO and IgM. As a non-limiting example can be mentioned single passage of more than one liter blood group B plasma with a. flow rate of ca 40 ml/minute through a column with ca 3 mikromol of blood group A trisaccharide per ml Sepharose 4 FF, and with a total product volume of 62 ml, and an average particle size of 90 µm, practically eliminated all antibodies reactive against blood group A. Similar result was obtained with blood group B product. The products were built according to 1.a. and 2.b. above from A- and B-trisaccharide-spacer and NHS-activated Sepharose^{R} 4 Fast Flow.

One or more columns can be used. The column volume is chosen for the purpose and can be for example of a size of, for example, 10 ml, 20 ml, 40 ml, 60 ml up to a size of for example 500 ml or higher depending on which volumes are desired to process. The column volume can be for example of a value between the given values. Different types of column houses of different dimensions can be used. The product according to the invention functions, as non-limiting example, in the type of column housing with the dimensions used for the product ImmunoSorba^{R}, (which has protein A as ligand bound to Matrix), which has an inner volume between the porous membranes of ca 62 ml (that is allows filling of 62 ml Material according to the invention).

When using Products according to the invention for treatment of plasma, can in general be used membranes which have a lower porosity and Matrix particles which have lower particle size as compared with the case when the Product is applied for treatment of whole blood. Thus, for example, in the case of treatment of plasma, membrane with porosity of for example 30 micrometer, or membrane with a porosity in the interval 20 till 40 micrometer, and particle size of Matrix of for example 90 micrometer, or Matrix of for example particle size in the interval 40 - 200 micrometer, can be used. When using Products according to the invention for treatment of whole blood, membrane with porosity of for example 30 micrometer or 70 micrometer, or membrane with a porosity in the interval 20 to 100 micrometer, can be used, and the particle size of the Matrix can be for example 150 micrometer, or the Matrix particle size can be for example in the interval 100 - 250 micrometer. The porosity is chosen by the expert and does not limit the scope of the invention.

The filling of the Product according to the invention in the column can be done using different principal methods. According to the invention, the Product can for example either be autoclaved first and thereafter be filled aseptically in the column, or the Product can, as an example of another preferred form of the invention, first be filled in the column and thereafter is the column with the Product autoclaved.

Non-limiting example of autoclaving is treatment in an autoclave of for example counterpressure type, which involves treatment under at least 20 minutes at 121 °C or higher and with for example water steam. Other conditions can be chosen by the expert from what is suitable, e.g. sterility and stability of the product. As an example can be mentioned that Saccharide-spacer-Matrix according to examples 1.a. and 2.b., obtained via coupling of the respective ligand to NHS-activated Sepharose 4FF, exhibits the same properties after autoclaving as before autoclaving concerning tested parameters such as antibody binding properties and other properties.

The column completely, or partially, filled with Material according to the invention, can for example be constructed in materials which allows for autoclaving (biocompatible plastic materials which can be autoclaved are commercially available, e.g. column housing and locks arc made of special polycarbonate, tubings of PYC or silicon material and rings of silicon) and/or for example to allow aseptic packing of Material according to the invention. Column house exists on the market for extracorporal blood treatment, e.g. Immunosorba. This allows for aseptic filling , but nor for autoclaving.

Non-limiting example of autoclavable column house is a column house built from autoclavable materials. Otherwise can the same principal design as on the market available column houses be used with two locks, both equipped with for example identical threads which are screwed, with help of the threads, outside and at both endings of a cylinder(house), which is equipped with matching threads at the two endings of the cylinder(house). Between each lock and cylinder is, before screwing together the locks and the cylinder, placed a porous membrane (that is two membranes and rings for each column), which allows for passage of plasma or whole blood but not for passage of the Material according to invention. Each membrane is mounted between the lock and the cylinder with for example a silicon ring with a fitting grove of ca the same or the same diameter as the cylinder. Every silicon ring has for example a grove which allows for fitting the circular membrane in the grove in the silicon ring. The membrane is mounted in the silicon ring and is placed between the lock and the ending of the cylinder, after which the lock is screwed on the cylinder as described above. The silicon ring with the membrane is therewith enclosed between the lock and the cylinder ending. The same procedure is carried out for the other ending of the cylinder. Each lock has a centrally placed hole with an elevation which allows for connecting a bio-compatible and autoclavable set of tubings equipped with connections of e.g. the Luer type for connection of other equipment used in extracorporal treatment.

Instead of above mentioned design where the locks and the cylinder are connected with threads, a clip mechanism can, for example, instead be used, where for example the locks are equipped with one or more clips (for example in each one of the locks there are 2, 3, 4, 5, 6, 7, 8, 9,10,11, 12, 13, 14, or more separate clips which are situated beside each other with a defined interspace), and the cylinder has on its outer side one or more protruding edge(s) placed at a distance, e.g- of 2, 3, 4, 5, or 6 mm from the top and from the bottom, respectively, of the cylinder. The clips can be protruding down from the locks and can for example be homogeneous, or each one be equipped with a cavity to allow for a larger flexibility without being broken. In this manner can the silicon ring with the membrane according to the above be placed between the lock and the respective cylinder ending, and the lock is thereafter pressed on the cylinder, whereupon the clips are pressed under de protruding edges on the cylinder and stays there, and the silicon ring with the porous membrane is consequently sealed between the lock and the cylinder.

In order to fill the so mounted column houses with Material according to the invention, the cylinder part can be equipped with a circular opening with a protruding part, which has threads, on the outer side of the cylinder to allow connection of a tubing used for filling of the material. After filling of the material into the column housing, a bio-compatible plug with threads which matches the threads of the protruding part on the cylinder, is mounted. In the center of the plug is a protruding tap which fits into the circular hole of the cylinder and which has a length which corresponds to the height of the protruding part In this manner an (almost) flat surface is achieved inside the cylinder at the circular opening.

For the autoclaving of a column filled with Material according to the invention and connected with a set of tubings, can an outer ring of e.g. PVC material, and with an inner diameter which corresponds with the outer diameter of the threads of each lock, be mounted around (the threads of) each lock before the autoclaving. This can be done in order to minimize any deformation of the threads under the autoclaving process. This principle has been successfully used under autoclaving of column filled with Material according to the above.

All mentioned components of the column house in a preferred example according to the invention with autoclavable column house, are autoclavable and biocompatible. Lock membrane, cylinder, plug, and tubing with luer coupling can be made of biocompatible plastic material.

Column house completely or partially filled with Material according to the invention and equipped with above mentioned closed tubing set and plug can be autoclaved. This facilitates according to the invention the achievement of sterility of the products according to the invention. With earlier methods sterile (aseptic) filling have been carried out, which are difficult to achieve.

## Claims

1. A column for treatment of whole blood or blood plasma, which is autoclavable and comprises
a column housing, having an inlet and an outlet for passage through the column of whole blood or blood plasma, said column housing containing a crosslinked matrix material to which at least one biologically active saccharide is covalently bound via at least one spacer, wherein the biologically active saccharide is a blood group determinant A or a blood group determinant B, and wherein the spacer is -O(CH₂)ₙPhNH-CO-(CH₂)ₘNH- or -O(CH₂)ₙPhNH-CO-(CH₂)ₘNH-CH₂-CH(OH)-CH₂-, wherein n is 0-4 and m is 1-7, or -O(CH₂)ₙNH-, wherein n is 1-7, or wherein said spacer is a peptide having the formula Gly-(Glu-Gly)ₙ-Glu, wherein n is 1-20, or a protein or a polysaccharide, bound to the biologically active saccharide via an O-glycosidically bound -O(CH₂)₂PhNH- or -O(CH₂),NH-, wherein n is 1-7, and wherein said spacer is bound to said matrix via -O-, and said column housing having a top end and a bottom end,
one lock attached to the top end of said column housing and one lock attached to the bottom end of said column housing, wherein each lock is provided with a centrally placed hole with an elevation which allows for connecting a biocompatible and autoclavable set of tubings equipped with connections for connection of other equipment used in extracorporal treatment,
a porous membrane, allowing for passage of blood plasma or whole blood, but not for passage of said cross-linked matrix material, and mounted with a ring between said ends of the column housing and each lock,
wherein the locks are attached to the top end and the bottom end, respectively, of the column housing in such a way that either
a) each lock and the top end and the bottom end of the column housing are equipped with matching threads, wherein said locks have been screwed on to the column housing on the outside and at the top end and the bottom end thereof, or that
b) each lock is provided with one or more clips protruding from the lock, and the top end and the bottom end of the column housing is provided on its outer side with one or more protruding edges placed at a distance from the top and from the bottom end, respectively, permitting the locks to be pressed on to said column housing, wherein said clips on each lock have been engaged with the protruding edges in said top end and bottom end, respectively.

2. The column according to claim 1, wherein the crosslinked matrix material is a polymer, plastic or a polysaccharide, preferably agarose.

3. The column according to claim 1, wherein the blood group determinant A is GalNAcα1-3(Fucα1-2)Galβ- and the blood group determinant B is Galα1-3(Fucα1-2)Galβ-, for extracorporeal removal of blood group A or blood group B antibodies, respectively, from whole blood or blood plasma.

4. The column according to claim 1 or 2, wherein the blood group determinant A is a blood group A determinant of type 1, 2, 3, or 4, and the blood group determinant B is a blood group B determinant of type 1, 2, 3, or 4, for extracorporeal removal of blood group A or blood group B antibodies, respectively, from whole blood or blood plasma.

5. The column according to claim 1, wherein each membrane is mounted between the locks and the ends of the column housing with a silicon ring.

6. The column according claims 1 or 5, wherein an outer ring, provided with an inner diameter corresponding with the outer diameter of the threads of each lock, is mounted around each lock in the case the locks are provided with threads.

7. The column according to any one of claims 1, 5, or 6, wherein the column housing is provided with a circular opening for the filling of said column housing with the crosslinked matrix material to which at least one biologically active saccharide is covalently bound via at least one spacer.

8. The column according to any one of claims 1 and 5-6, wherein all components thereof are made of biocompatible materials.

9. The column according to any one of claims 1 and 5-8, wherein each membrane in the case of treatment of blood plasma has a porosity of 20-40 µm and each matrix has a particle size of 40-200 µm.

10. The column according to one of claims 1 and 5-9, wherein each membrane in the case of treatment of whole blood has a porosity of 20-100 µm and each matrix has a particle size of 100-250 µm.

11. The column according to any one of claims 1, and 5-10, wherein said column housing has an inner volume of 10 ml to 500 ml.

12. The column according to any one of claims 1, and 5-11, wherein each lock is provided with a tubing with a connection means of Luer type for connection of other equipment used in extracorporeal treatment.

13. The column according to claim 6, wherein the outer ring mounted around the locks at the top end and bottom end are made of a PVC material.

14. The column according to claims 1 and 5-13, wherein the column housing and the locks are made of a biocompatible polycarbonate and the tubings are made of PVC or a silicon material.

15. The column according to claims 1 and 5-14, wherein it has been autoclaved.

16. The column according to claim 15, wherein it has been autoclaved using water steam for at least 20 minutes, and at a temperature of at least 121°C.

17. The column according to claim 15, wherein it is completely or partially filled, and is equipped with a closed tubing set and a plug.

18. A column according to claim 1, wherein the protein is serum albumin.

19. A column according to claim 1, wherein the polysaccharide is dextran.

20. A method for filling the column according to any one of the preceding claims, comprising connecting the column housing with a tubing at a circular opening having a protruding part with threads on the outer side of the column of the column housing, filling the crosslinked matrix with at least one biologically active saccharide covalently bound via at least one spacer into the column housing through said opening and mounting in said opening a biocompatible plug with threads which matches threads of a protruding part of the column housing around said opening and having a protruding tap in the center of the plug which fits into the circular hole of the column.

21. A product represented by the structure:
saccharide-spacer-O-matrix, wherein at least one saccharide is covalently bound to the matrix via at least one spacer, and wherein the saccharide is biologically active and is a blood group determinant A or a blood group determinant B;
wherein the matrix is a plastic or crosslinked agarose, and wherein the spacer is -O(CH₂)ₙPhNH-CO-(CH₂)ₘNH-, and -O(CH₂)ₙPhNH-CO-(CH₂)ₘNH-CH₂-CH(OH)-CH₂-, wherein n is 0 to 4 and m is 1-7.

22. Method for extracorporeal removal of blood group A and blood group B antibodies from whole blood or blood plasma, wherein said whole blood or blood plasma is brought to pass through a column according to any one of claims 1-17.

23. Use of a column according to any one of claims 1-19 for extracorporeal removal of blood group A and blood group B antibodies from whole blood or blood plasma.

24. Use of a product according to claim 21 in a column according to any one of claims 1-19 for extracorporeal removal of blood group A and blood group B antibodies from whole blood or blood plasma.

## Patentansprüche

1. Eine Säule zur Behandlung von Vollblut oder Blutplasma, welche autoklavierbar ist und umfasst
ein Säulengehäuse mit einem Einlass und einem Auslass für die Passage des Vollbluts oder Blutplasmas durch die Säule, wobei das Säulengehäuse ein vernetztes Matrixmaterial enthält, an welches mindestens ein biologisch aktives Saccharid mittels mindestens einem Spacer kovalent gebunden ist, worin das biologisch aktive Saccharid ein Blutgruppe-bestimmender Faktor A oder ein Blutgruppe-bestimmender Faktor B ist und worin der Spacer -O(CH₂)ₙPhNH-CO-(CH₂)ₘNH- oder -O(CH₂)ₙPhNH-CO-(CH₂)ₘNH-CH₂-CH(OH)-CH₂- ist, worin n 0 bis 4 ist und m 1 bis 7 ist, oder-O(CH₂)ₙNH-, worin n 1 bis 7 ist, oder worin dieser Spacer ein Peptid mit der Formel Gly-(Glu-Gly),-Glu ist, worin n 1 bis 20 ist oder ein Protein oder ein Polysaccharid, die an das biologisch aktive Saccharid mittels eines O-glycosidisch gebundenen O(CH₂)₂PhNH- oder -O(CH₂)NH-gebunden sind, wann n 1 bis 7 ist, und worin der Spacer an die Matrix mittels -O- gebunden ist, und worin das Säulengehäuse ein oberes Ende und ein unteres Ende aufweist,
einen Verschluss, der an das obere Ende des Säulengehauses angebracht ist und einen Verschluss, der an dem unteren Ende des Säulengehäuses angebracht ist, wobei jeder Verschluss mit einem zentral platzierten Loch ausgestattet ist mit einer Erhöhung, welche erlaubt, ein biokompatibles und autoklavierbares Set von Schläuchen, das mit Anschlüssen für das Anschließen weiterer Ausrüstungsgegenstande, die in extrakorporaler Behandlung verwender werden, ausgestattet ist, anzuschließen,
eine poröse Membran, die die Passage des Blutplasmas oder Vollblut erlaubt, aber nicht die Passage des vernetzten Matrixmaterials und weiche mit einem Ring zwischen den Enden des Säulengehäuses und jedem Verschluss aufgebracht ist,
worin die Verschlüsse an dem oberen und unteren Ende des Säulengehäuses so angebracht sind, dass entweder
(a) jeder Verschluss und das obere und untere Ende des Säulengehäuses mit passenden Gewinden ausgestattet sind, worin die verschlüsse auf das Säulengehäuse an der Außenseite und an dem oberen und unteren Ende davon geschraubt worden sind, oder dass
(b) jeder Verschluss mit einer oder mehreren Klammern ausgestattet ist, die aus dem Verschluss hervorstehen, und das obere Ende und das untere Ende des Säulengehäuses auf seiner Außenseite mit einer oder mehreren hervorstehenden Kanten ausgestattet ist, die jeweils in einer Entfernung vom oberen und unteren Ende plaziert werden, die erlaubt, dass die Verschlüsse auf das Säulengehäuse gepresst werden, worin die Klammern auf jedem Verschluss in die vorstehenden Kanten der oberen und unteren Enden eingerastet sind.

2. Die Säule gemäß Anspruch 1, worin das vernetzte Matrix-Material ein Polymer, Plastik oder ein Polysaccharid, bevorzugt Agarose, ist.

3. Die Säule gemäß Anspruch 1, worin der B!utgruppe-bestimmende Faktor A Ga!-NAcα1-3(Fuca1-2)Galβ- und der Blutgruppe-bestimmende Faktor B Galα1-3(Fucα1-2)Galβ- ist, jeweils zur extrakorporalen Entfernung von Antikörpern der Blutgruppe A oder Antikörpern der Blutgruppe B aus Vollblut oder Blutplasma.

4. Die Säule gemäß Anspruch 1 oder 2, worin der Blutgruppe-bestimmende Faktor A ein Blutgruppe A bestimmender Faktor des Typs 1, 2, 3 oder 4 ist und der Blutgruppe-bestimmende Faktor B ein Blutgruppe 8 bestimmender Faktor des Typs 1,2, 3 oder 4 ist, jeweils zur extrakorporalen Entfernung der Blutgruppe A oder Blutgruppe B aus Vollblut, oder Blutplasma,

5. Die Säule gemäß Anspruch 1, worin jede Membran zwischen den Verschlüssen und den Enden des Säulengehäuses mit einem Siliconring montiert ist.

6. Die Säule gemäß Ansprüchen 1 oder 5, worin ein äußerer Ring, der mit einem inneren Durchmesser ausgestattet ist, der mit dem äußeren Durchmesser des Gewindes jedes Verschlusses korrespondiert, um jeden Verschluss herum befestigt ist für den Fall, dass die Verschlüsse mit Gewinden ausgestattet sind.

7. Die Säule gemäß einem der Ansprüche 1,5 oder 6, worin das Säulengehäuse mit einer runden Öffnung für das Füllen des Säulengehäuses mit dem vernetzten Matrix-Material, an welches mindestens ein biologisch aktives Saccharid mittels mindestens einem Spacer kovalent gebunden ist, ausgestattet ist

8. Die Säule gemäß einem der Ansprüche 1 und 5 bis 6, worin alle ihre Bestandteile aus biokompatiblen Materialien gemacht sind.

9. Die Säule gemäß einem der Ansprüche 1 und 5 bis 8, worin jede Membran in dem Falle einer Behandlung von Blutplasma eine Porosität von 20-40 µm aufweist und jede Matrix eine Partikelgröße von 40-200 µm aufweist.

10. Die Säule gemäß einem der Ansprüche 1 und 5 bis 9, worin jede Membran in dem Falle der Behandlung von Vollblut eine Porosität von 20-100 µm aufweist und jede Matrix eine Partikelgröße von 100-250 µm aufweist.

11. Die Säule gemäß einem der Ansprüche 1 und 5 bis 10, worin das Säulengehäuse ein inneres Volumen von 10 ml bis 500 ml hat.

12. Die Säule gemäß einem der Ansprüche 1 und 5 bis 11, worin jeder Verschluss mit einem Schlauch mit einem Anschluss des Luer-Typs zum Anschließen weiterer Ausrüstung, die in extrakorporaler Behandlung verwendet wird, ausgestattet ist.

13. Die Säule gemäß Anspruch 6, worin der äußere Ring, der um die Verschlüsse am oberen und unteren Ende montiert ist, aus einem PVC-Material gemacht ist.

14. Die Säule gemäß Ansprüchen 1 und 5 bis 13, worin das Säulengehäuse und die Verschlüsse aus einem biokompatiblen Polycarbonat und die Schläuche aus PVC oder einem Silikon-Material gemacht sind.

15. Die Säule gemäß Ansprüchen1 1 und 5 bis 14, worin sie autoklaviert wurde.

16. Die Säule gemäß Anspruch 15, worin sie unter Verwendung von Wasserdampf für mindestens 20 Minuten und bei einer Temperatur von mindestens 121 °C autoklaviert wurde.

17. Die Säule gemäß Anspruch 15, worin sie ganz oder teilweise gefügt ist und ausgestattet ist mit einem geschlossenen Schlauchset und einem Verschlussstopfen

18. Die Säule gemäß Anspruch 1, worin das Protein Serumalbumin ist.

19. Die Säule gemäß Anspruch 1, worin das Polysaccharid Dextran ist.

20. Ein Verfahren zum Befüllen der Säule gemäß einem der vorangehenden Ansprüche, umfassend das Verbinden des Säulengehäuses mit einem Schlauch an einer runden Öffnung mit einem hervorstehenden Teil mit Gewinden an der äußeren Seite der Säule des Säulengehäuses, Befüllen des vernetzten Materials mit mindestens einem biologisch aktiven Saccharid, das mit mindestens einem Spacer verbunden ist, in das Säutengehäuse durch diese Öffnung und Montieren eines biokompatiblen Verschlussstopfers mit Gewinden in diese Öffnung, wobei die Gewinde mit den Gewinden des hervorstehenden Teils des Säulengehäuses um die Öffnung harmonieren und der einen hervorstehenden Hahn in der Mitte des Verschlussstopfens ausweist, weicher !n das runde Loch der Säule passt.

21. Ein Produkt, dargestellt durch die Struktur:
Saccharid-Spacer-O-Matrix,
worin mindestens ein Saccharid kovalent an die Matrix mittels mindestens einem Spacer gebunden ist und worin das Saccharid biologisch aktiv ist und ein Blutgruppe-bestimmender Faktor A oder ein Blutgruppe-bestimmender Faktor B ist,
worin die Matrix ein Plastik oder vernetzte Agarose ist und wenn der Spacer -O(CH₂)ₙPhNH-O-(CH₂)ₘNH- und -O(CH₂)ₘPhNH-CO-(CH₂)NH-OH CH₂- ist, worin n 0-4 und m 1-7 ist.

22. Verfahren für das extrakorporale Entfernen von Antikörpern der Blutgruppe A und Blutgruppe B aus Vollblut oder Blutplasma, worin das Vollblut oder Blutplasma dazu gebracht wird, durch eine Säule gemäß einem der Anspruche 1 bis 17 zu passieren,

23. Verwendung einer Säule gemäß einem der Ansprüche 1 bis 19 zur extrakorporalen Entfernung von Antikörpern der Blutgruppe A und Blutgruppe B aus Vollblut oder Blutplasma.

24. Verwendung eines Produkts gemäß Anspruch 21 in einer Säule .gemäß einem der Anspruche 1 bis 19 zur extrakorporalen Entfernung von Antikörpern der Blutgruppe A und Blutgruppe B aus Vollblut oder Blutplasma,

## Revendications

1. Colonne pour le traitement du sang total ou du plasma sanguin, qui est autoclavable et comprend
un boîtier de colonne, ayant une entrée et une sortie pour le passage dans la colonne du sang total ou du plasma sanguin, ledit boîtier de colonne contenant une matière de matrice réticulée à laquelle au moins un saccharide biologiquement actif est lié de manière covalente via au moins un espaceur, où le saccharide biologiquement actif est un déterminant de groupe sanguin A ou un déterminant du groupe sanguin B, et où l'espaceur est -O(CH₂)ₙPhNH-CO-(CH₂)ₘNH- ou -O(CH₂)ₙPhNH-CO-(CH₂)ₘNH-CH2-CH(OH)-CH₂-, où n est 0-4 et m est 1-7, ou -O(CH₂)ₙ-NH-, où n est 1-7, ou bien où ledit espaceur est un peptide ayant la formule Gly-(Glu-Gly)ₙ-Glu, où n est 1-20, ou une protéine ou un polysaccharide, lié au saccharide biologiquement actif via un -O(CH₂)₂PhNH- ou -O(CH₂)ₙNH- lié de manière O-glycosidique, où n est 1-7, et où ledit espaceur est lié à ladite matrice via -O-, et ledit boîtier de colonne ayant une extrémité supérieure et une extrémité inférieure,
un raccord fixé à l'extrémité supérieure dudit boîtier de colonne et un raccord fixé à l'extrémité inférieure dudit boîtier de colonne, où chaque raccord est muni d'un trou placé au centre avec une élévation qui permet la connexion d'un ensemble biocompatible et autoclavabe de tubes équipés de connexions pour la connexion d'autres appareillages utilisés dans un traitement extracorporel,
une membrane poreuse, permettant le passage du plasma sanguin ou du sang total, mais pas le passage de ladite matière de matrice réticulée, et montée avec un anneau entre lesdites extrémités du boîtier de colonne et chaque raccord,
où les raccords sont fixés à l'extrémité supérieure et à l'extrémité inférieure, respectivement, du boîtier de colonne de telle manière que
a) chaque raccord et extrémité supérieure et l'extrémité inférieure du boîtier de colonne sont équipés de filets appariés, où lesdits raccords ont été vissés sur le boîtier de colonne sur l'extérieur et à l'extrémité supérieure et à l'extrémité inférieure de celui-ci, ou bien
b) chaque raccord est muni d'une ou plusieurs attaches faisant saillie du raccord, et l'extrémité supérieure et l'extrémité inférieure du boîtier de colonne sont munies sur leur côté externe d'un ou plusieurs bords en saillie placés à une distance de l'extrémité supérieure et de l'extrémité inférieure, respectivement, permettant aux raccords d'être pressés sur ledit boîtier de colonne, où lesdites attaches sur chaque raccord ont été amenées à coopérer avec les bords en saillie dans ladite extrémité supérieure et ladite extrémité inférieure, respectivement,

2. Colonne selon la revendication 1, où la matière de matrice réticulée est un polymère, une matière plastique ou un polysaccharide, de préférence l'agarose.

3. Colonne selon la revendication 1, où le déterminant de groupe sanguin A est GalNAcα1-3(Fucα1-2)Galβ- et le déterminant de groupe sanguin B est Galα1-3(Fucα1-2)Galβ-, pour le retrait extracorporel d'anticorps de groupe sanguin A ou de groupe sanguin B, respectivement, du sang total ou du plasma sanguin.

4. Colonne selon la revendication 1 ou 2, où le déterminant de groupe sanguin A est un déterminant de groupe sanguin A de type 1, 2, 3 ou 4, et le déterminant de groupe sanguin B est un déterminant de groupe sanguin B de type 1, 2, 3 ou 4, pour le retrait extracorporel d'anticorps de groupe sanguin A ou de groupe sanguin B, respectivement, du sang total ou du plasma sanguin,

5. Colonne selon la revendication 1, où chaque membrane est montée entre les raccords et les extrémités du boîtier de colonne avec un anneau en silicone,

6. Colonne selon les revendications 1 ou 5, où un anneau externe, muni d'un diamètre interne correspondant au diamètre externe des mets de chaque raccord, est monté autour de chaque raccord dans le cas où les raccords sont munis de filets.

7. Colonne selon l'une quelconque des revendications 1, 5 ou 6, où le boîtier de colonne est muni d'une ouverture circulaire pour le remplissage dudit boîtier de colonne avec la matière de matrice réticulée à laquelle au moins un saccharide biologiquement actif est lié de manière covalente via au moins un espaceur.

8. Colonne selon l'une quelconque des revendications 1 et 5-6, où tous ses composants sont en matières biocompatibles.

9. Colonne selon l'une quelconque des revendications 1 et 5-8, où chaque membrane dans le cas d'un traitement du plasma sanguin a une porosité de 20-40 µm et chaque matrice a une taille de particule de 40-200 µm,

10. Colonne selon l'une des revendications 1 et 5-9, où chaque membrane dans le cas d'un traitement du sang total a une porosité de 20-100 µm et chaque matrice a une taille de particule de 100-2.50 µm.

11. Colonne selon l'une quelconque des revendications 1 et 5-10, où ledit bottier de colonne a un volume interne de 10 ml à 500 ml.

12. Colonne selon l'une quelconque des revendications 1 et 5-11, où chaque raccord est muni d'un tube avec un moyen de connexion de type Luer pour la connexion d'autres appareillages utilisés dans un traitement extracorporel.

13. Colonne selon la revendication 6, où l'anneau externe monté autour des raccords à S'extrémité supérieure et à l'extrémité inférieure sont en une matière de type PVC.

14. Colonne selon les revendications 1 et 5-13, où le boîtier de colonne et les raccords sont en un polycarbonate biocompatible et les tubes sont en PVC ou une matière du type silicone.

15. Colonne selon les revendications 1 et 5-14, où elle a été autoclavée.

16. Colonne selon la revendication 15, où elle a été autoclavée avec de la vapeur d'eau pendant au moins 20 min et à une température d'au moins 121°C.

17. Colonne selon la revendication 15, où elle est complètement ou partiellement remplie et est équipée d'un ensemble de tubes fermé et d'un bouchon.

18. Colonne selon la revendication 1, où la protéine est la sérumalbumine.

19. Colonne selon la revendication 1, où le polysaccharide est le dextrane.

20. Procédé pour remplir la colonne selon l'une quelconque des revendications précédentes, comprenant la connexion du boîtier de colonne avec un tube au niveau d'une ouverture circulaire ayant une partie en saillie avec des filets sur le côté externe de la colonne du boîtier de colonne, le remplissage du boîtier de colonne avec la matrice réticulée avec au moins un saccharide biologiquement actif lié de manière covalente via au moins un espaceur par ladite ouverture et le montage dans ladite ouverture d'un bouchon biocompatible avec des filets qui coïncident avec les filets d'une partie en saillie du boîtier de colonne autour de ladite ouverture et ayant un robinet en saillie au centre du bouchon qui s'adapte dans le trou circulaire de la colonne.

21. Produit représenté par la structure : saccharide-espaceur-O-matrice, où au moins un saccharide est lié de manière covalente à la matrice via au moins un espaceur, et où le saccharide est biologiquement actif et est un déterminant de groupe sanguin A ou un déterminant de groupe sanguin B ;
où la matrice est une matière plastique ou un agarose réticulé, et où l'espaceur est -O(CH₂)ₙPhNH-CO-(CH₂)ₘNH- et -O(CH₂)ₙPₕNH-CO-(CH₂)ₘNH-CH₂-CH(OH)-CH₂-, où n est 0 à 4 et m est 1-7.

22. Procédé pour le retrait extracorporel d'anticorps de groupe sanguin A et de groupe sanguin B du sang total ou du plasma sanguin, où ledit sang total ou ledit plasma sanguin est amené à passer dans une colonne selon l'une quelconque des revendications 1-17.

23. Utilisation d'une colonne selon l'une quelconque des revendications 1-19 pour le retrait extracorporel d'anticorps de groupe sanguin A et de groupe sanguin B du sang total ou du plasma sanguin,

24. Utilisation d'un produit selon la revendication 21 dans une colonne selon l'une quelconque des revendications 1-19 pour le retrait extracorporel d'anticorps de groupe sanguin A et de groupe sanguin B du sang total ou du plasma sanguin.
